# EUROPÄISCHE PATENTSCHRIFT

(11) **EP 0 510 339 B1**
(45) Veröffentlichungstag und Bekanntmachung des Hinweises auf die Patenterteilung: **06.08.1997**
(21) Anmeldenummer: 92104046.5
(22) Anmeldetag: 10.03.1992
(51) Int. Cl.: G01N 21/25, G01N 33/49, G01N 21/03

(54) **Vorrichtung zur photometrischen Bestimmung des Gerinnungsverhaltens von Blut, Blutplasma oder dergleichen**
Photometrical device for the determination of clotting of blood, blood plasma and similar
Dispositif photométrique pour déterminer la coagulation du sang, du plasma sanguin etc.

(30) Priorität: 24.04.1991 DE 4113330
(43) Veröffentlichungstag der Anmeldung: 28.10.1992
(73) Patentinhaber: HEINRICH AMELUNG GmbH, D-32657 Lemgo (DE)
(72) Erfinder: Eggl, Wilfried, W-4920 Lemgo (DE); Amelung, Rolf, W-4920 Lemgo (DE)
(74) Vertreter: Stracke, Alexander, Dipl.-Ing.

(56) Entgegenhaltungen:
- WO-A-83/00228
- DE-A- 2 531 136
- DE-U- 7 719 078
- US-A- 4 497 774

## Beschreibung

Die vorliegende Erfindung betrifft eine Vorrichtung zur photometrischen Bestimmung des Gerinnungsverhaltens von Blut, Blutpalsma od. dgl. gemäß dem Oberbegriff des Anspruchs 1. Eine solche Vorrichtung ist aus dem Dokument WO-A-83 00228 bekannt.

Zur photometrischen Bestimmung des Gerinnungsverhaltens von Blut oder Blutplasma ist es bekannt, eine mit einer entsprechenden Probe versehene, durchsichtige Küvette in die Küvettenaufnahme eines Küvettenhalters einzustecken, wobei der Querschnitt der Küvettenaufnahme dem der Küvette angepaßt ist, also vorzugsweise rechteckig gestaltet ist. Die photometrische Meßeinrichtung aus einem im Bereich der einen Schmalseite angeordneten Sender und einem im Bereich der anderen Schmalseite vorgesehenen Empfänger.

Bislang wird die Küvette nach der photometrischen Messung aus der Küvettenaufnahme herausgezogen. Dies kann manuell geschehen. Es sind aber auch Beschickungsautomaten bekannt, mit denen ein automatisches Einstecken und Herausziehen erfolgt.

In jedem Fall ist als nachteilig anzusehen, daß durch den Arbeitsgang des Herausziehens der Küvette aus der Küvettenaufnahme eine relativ lange Zeit verstreicht, bis eine erneute Lichtwellenmessung durchgeführt werden kann.

Insbesondere bei der Messung von vielen Proben, wie sie beispielsweise in medizinischen Labors durchgeführt werden, ergeben sich erhebliche wirtschaftliche Nachteile, da die Vorrichtung nicht in optimaler Weise genutzt werden kann.

Besonders gravierend macht sich dieser Nachteil bemerkbar, wenn der Küvettenhalter eine Vielzahl von Küvettenaufnahmen aufweist, die automatisch bestückt werden, wobei dann bei jeder Entnahme der Küvette durch z. B. einen Greifarm, ein Rückfahrweg von diesem Greifarm zurückgelegt werden muß, bis die entsprechende Küvette abgelegt werden kann.
Die Zeit, die der Greifer benötigt, um diese Rückfahrstrecke zurückzulegen, addiert sich noch zur eigentlichen Entnahmezeit, so daß sich daraus ein einem effektiven Betrieb entgegenstehender Faktor ergibt.

Andererseits birgt die manuelle Entnahme der Küvette gewisse Risiken, da es bei deren Entsorgung, das heißt beim Ablegen in geeigneten Behältern, durchaus zum Kontakt des Bedienungspersonals mit dem Küvetteninhalt kommen kann.

Der vorliegenden Erfindung liegt daher die Aufgabe zugrunde, eine Vorrichtung der gattungsgemäßen Art so zu gestalten, daß das Entfernen der Küvette aus der Küvettenaufnahme schneller und einfacher möglich ist und so der Betrieb der Vorrichtung wirtschaftlicher wird.

Diese Aufgabe wird erfindungsgemäß durch die im kennzeichnenden Teil des Anspruchs 1 genannten Merkmale gelöst.

Diese konstruktiven Maßnahmen bringen mit sich, daß praktisch eine Zeit zum Entfernen der Küvetten aus der Küvettenaufnahme nicht mehr notwendig ist. Im Prinzip wird nämlich die Küvette, deren Probe gemessen wurde und die in der Küvettenaufnahme arretiert ist, durch die neu in die Küvettenaufnahme einzubringende Küvette ausgestoßen, wobei die entfernte Küvette zweckmäßigerweise in einen Behälter fällt, der unterhalb des Küvettenhalters plaziert ist.

Dabei ist es sinnvoll, wenn die auszustoßende Küvette aus der Arretierung gelöst wird, bevor die einzulegende Küvette ihre arretierte Endstellung erreicht hat.

Die Haltemittel, mit der die Küvette arretierbar ist, können beispielsweise durch Federn gebildet sein, mit denen die Küvette an die Wandung der Küvettenaufnahme anpreßbar ist, wobei dann die Küvette durch Reibschluß gehalten wird.
Selbstverständlich ist aber auch die Ausbildung der Haltemittel in einer anderen Art und Weise denkbar.

Ein weiterer Vorteil der Erfindung liegt darin, daß nunmehr der photometrischen Meßeinrichtung eine Inkubationseinrichtung vorgeschaltet sein kann, in der die zu messende Probe erwärmt und auf einer bestimmten Temperatur gehalten wird und die beispielsweise unmittelbar oberhalb der Küvettenaufnahme angeordnet ist.
Dabei würde die Inkubationseinrichtung einen der Küvettenaufnahme entsprechende Schacht aufweisen, der gleichfalls mit Haltemitteln versehen ist, so daß nach Beendigung der Inkubationszeit, die länger ist als die Zeit der photometrischen Messung der darunterliegenden Küvette, die erwärmte Küvette unter Ausstoßen der Küvette, die in der Küvettenaufnahme gehalten wird, in eine Position gebracht wird, die eine photometrische Messung ermöglicht.

Denkbar ist aber auch, die entsprechende Küvette einer separaten Inkubationseinrichtung zu entnehmen und in die Küvettenaufnahme einzustecken.

In jedem Fall wird in die neu einzubringende Küvette die darunterliegende ausgestoßen, wobei zweckmäßigerweise das Abmaß des Bodenbereiches dem Abmaß des Kopfbereiches entspricht, so daß kein Verklemmen oder Verhaken zweier Küvetten möglich ist.

Weitere vorteilhafte Ausgestaltungen der Erfindung sind in den Unteransprüchen gekennzeichnet.

Ein Ausführungsbeispiel der Erfindung wird nachfolgend anhand der beigefügten Zeichnungen beschrieben.

Es zeigen:
- Fig. 1: eine stark schematisierte schaubildliche Schnittdarstellung einer erfindungsgemäßen Vorrichtung,
- Fig. 2: einen Teillängsschnitt durch eine Küvettenaufnahme der Vorrichtung.

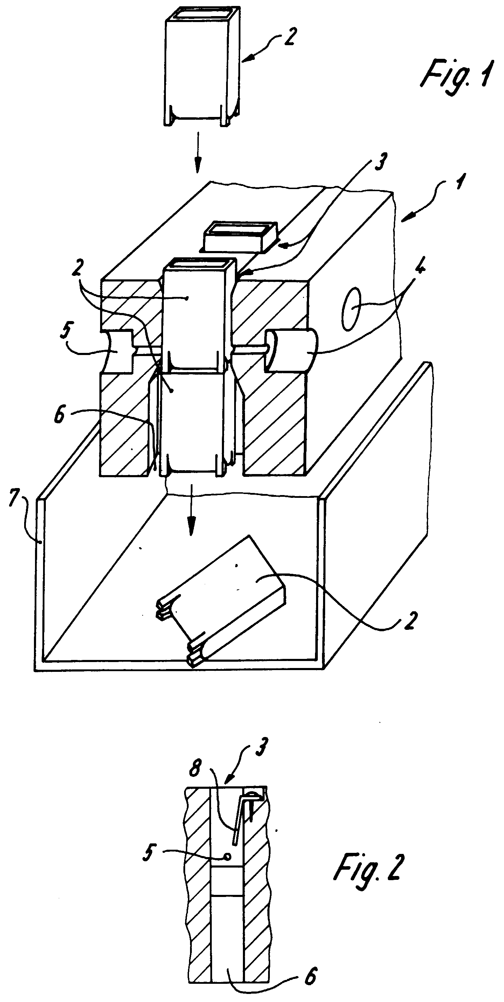

In der Figur 1 ist eine Vorrichtung zur photometrischen Bestimmung des Gerinnungsverhaltens von Blut, Blutplasma od. dgl. dargestellt, die einen Küvettenhalter 1 aufweist, in dem mehrere abständig zueinander angeordnete Küvettenaufnahmen 3 vorgesehen sind, die einen dem Querschnitt einer quaderförmigen Küvette 2 angepaßten rechteckigen Querschnitt aufweisen und die stirnseitig offen sind.

Dabei ist jede Küvettenaufnahme 3 von oben her mit einer Küvette 2 zu bestücken, was manuell, aber auch durch einen nicht dargestellten, einen Greifer aufweisenden Automaten erfolgen kann.

Jede Küvettenaufnahme 3 geht in einen unterhalb angeordneten Schacht 6 über, dessen Querschnittsabmaße größer sind als die entsprechenden Querschnittsabmaße der Küvette 2.

In dem Küvettenhalter 1 sind eine Senderaufnahme 4 und eine Empfängeraufnahme 5 einer photometrischen Meßeintung vorgesehen, wobei die Senderaufnahme 4 bzw. die Empfängeraufnahme 5 auf den sich gegenüberliegenden Schmalseiten der Küvettenaufnahme 3 in diese münden. Hier können die für eine photometrische Messung erforderlichen Teile untergebracht werden.

Unter dem Küvettenhalter 1 ist ein Sammelbehälter 7 angeordnet, in dem die durch den Schacht 6 ausgestoßenen Küvetten 2 gesammelt werden. Dieser Sammelbehälter 7 kann so ausgestaltet und angeordnet sein, daß er bei Bedarf entnehmbar und die gesammelten Küvetten 2 ohne weiteres entsorgbar sind.

Wie in der Figur 2 sehr deutlich zu erkennen ist, ist an einer Breitseite der Küvettenaufnahme 3 ein Haltemittel in Form einer Blattfeder 8 vorgesehen, die in das innere der Küvettenaufnahme 3 ragt.

Mit Hilfe dieser Blattfeder 8 wird die in die Küvettenaufnahme 3 gesteckte Küvette 2 gegen die gegenüberliegende Wandung der Küvettenaufnahme 3 gedrückt und so durch Reibschluß in einer bestimmten Stellung arretiert, so daß eine uneingeschränkte photometrische Messung möglich ist.

Dabei ist die Blattfeder 8 so angeordnet, daß eine gehaltene Küvette 2 bereits dann aus dem Wirkbereich der Blattfeder 8 gelangt, wenn eine von oben eingeführte neue Küvette 2 noch nicht ihre arretierte Endlage erreicht hat. Das heißt, vor Erreichen der Endstellung einer neuen Küvette 2 fällt die auszustoßende Küvette 2 bereits durch den Schacht 6 in den Sammelbehälter 7. Statt durch eine Blattfeder kann die Küvette 2 ausschließlich durch Reibschluß in der Küvettenaufnahme gehalten werden, wobei allerdings deren Querschnittsabmaße sehr genau denen der Küvette 2 entsprechen müssen. Um dabei ein einwandfreies Ausstoßen der Küvette 2 zu garantieren, ist es wichtig, daß die Tiefe der Küvettenaufnahme 3 kleiner ist als die zugeordnete Höhe der Küvette 2.

## Patentansprüche

1. Vorrichtung zur photometrischen Bestimmung des Gerinnungsverhaltens von Blut, Blutplasma od. dgl., mit einem Küvettenhalter, der mit mindestens
einer Küvettenaufnahme versehen ist, in die eine das Blut aufnehmende, durchsichtige, im Querschnitt vorzugsweise rechteckige Küvette von oben her einsteckbar ist, wobei im Bereich der Küvettenaufnahme eine Lichtwellenmeßeinrichtung angeordnet ist, und mit Haltemitteln in der Küvettenaufnahme (3), mit denen die Küvette (2) arretierbar ist **dadurch gekennzeichnet**, daß die Küvettenaufnahme (3) auf ihrer Unterseite eine Durchlaßöffnung aufweist.

2. Vorrichtung nach Anspruch 1, dadurch gekennzeichnet, daß die Küvettenaufnahme (3) in ihren Querschnittsabmaßen etwa den Querschnittsabmaßen der Küvette (2) entspricht.

3. Vorrichtung nach Anspruch 1, dadurch gekennzeichnet, daß sich an die Durchlaßöffnung ein Schacht (6) anschließt, der in seinen Querschnittsabmaßen größer ist als die Querschnittsabmaße der Küvette (2).

4. Vorrichtung nach Anspruch 1, dadurch gekennzeichnet, daß als Haltemittel eine Blattfeder (8) vorgesehen ist, die in die Küvettenaufnahme (3) hineinragt.

5. Vorrichtung nach Anspruch 1, dadurch gekennzeichnet, daß die Tiefe der Küvettenaufnahme (3) kleiner ist als die von einer Längskante bestimmte zugeordnete Höhe der Küvette (2).

6. Vorrichtung nach Anspruch 1, dadurch gekennzeichnet, daß unterhalb des Küvettenhalters (1) ein abnehmbarer Sammelbehälter (7) angeordnet ist.

7. Vorrichtung nach Anspruch 1, dadurch gekennzeichnet, daß oberhalb des Küvettenhalters (1) eine Inkubationseinrichtung vorgesehen ist, die Inkubationsschächte aufweist, von denen jeweils einer einer Küvettenaufnahme (3) zugeordnet ist, wobei der Inkubationsschacht und die zugeordnete Küvettenaufnahme eine gemeinsame Durchgangsöffnung aufweisen.

## Claims

1. Apparatus for the photometric determination of the coagulation behaviour of blood, blood plasma or the like, having a vessel holder provided with at least one vessel receiving means into which a transparent vessel which is preferably of rectangular cross-section for receiving the blood can be inserted from above, wherein arranged in the region of the vessel receiving means is a light wave measuring device, and having holding means in the vessel receiving means (3), with which the vessel (2) can be arrrested, characterised in that the vessel receiving means (3) has a passage opening on its underside.

2. Apparatus according to claim 1 characterised in that the vessel receiving means (3) approximately corresponds in its cross-sectional dimensions to the cross-sectional dimensions of the vessel (2).

3. Apparatus according to claim 1 characterised in that adjoining the passage opening is a shaft (6) which is larger in its cross-sectional dimensions than the cross-sectional dimensions of the vessel (2).

4. Apparatus according to claim 1 characterised in that a leaf spring (8) which projects into the vessel receiving means (3) is provided as the holding means.

5. Apparatus according to claim 1 characterised in that the depth of the vessel receiving means (3) is less than the associated height of the vessel (2) which is determined by a longitudinal edge.

6. Apparatus according to claim 1 characterised in that a removable collecting container (7) is arranged beneath the vessel holder (1).

7. Apparatus according to claim 1 characterised in that provided above the vessel holder (1) is an incubation device having incubation shafts, each of which is associated with a respective vessel receiving means (3), wherein the incubation shaft and the associated vessel receiving means have a common passage opening.

## Revendications

1. Dispositif de détermination photométrique de la coagulation du sang, du plasma sanguin ou similaire, avec un porte-cuvettes, qui est pourvu d'au moins un logement de cuvette, dans lequel une cuvette de section de préférence rectangulaire, transparente, recevant le sang, peut être insérée à partir du haut, un dispositif de mesure d'ondes de lumière étant placé dans la zone du logement de cuvette, et avec des moyens de maintien dans le logement de cuvette (3) avec lesquels la cuvette (2) peut être bloquée, caractérisé en ce que le logement de cuvette (3) présente sur sa face inférieure une ouverture de passage.

2. Dispositif selon la revendication 1, caractérisé en ce que le logement de cuvette (3) correspond dans ses dimensions de section transversale à peu près aux dimensions de section transversale de la cuvette (2).

3. Dispositif selon la revendication 1, caractérisé en ce qu'à l'ouverture de passage fait suite un puits (6), dont les dimensions de section transversale sont supérieures aux dimensions de section transversale de la cuvette (2).

4. Dispositif selon la revendication 1, caractérisé en ce qu'il est prévu comme moyens de maintien un ressort à lame (8), qui pénètre dans le logement de cuvette (3).

5. Dispositif selon la revendication 1, caractérisé en ce que la profondeur du logement de cuvette (3) est inférieure à la hauteur correspondante, déterminée par un côté longitudinal, de la cuvette (2).

6. Dispositif selon la revendication 1, caractérisé en ce qu'au-dessous du porte-cuvettes (1) est placé un récipient de collecte (7) amovible.

7. Dispositif selon la revendication 1, caractérisé en ce qu'au-dessus du porte-cuvettes (1) est prévu un dispositif d'incubation, qui présente des puits d'incubation, chacun desquels est affecté à un logement de cuvette (3), le puits d'incubation et le logement de cuvette correspondant présentant une ouverture de passage commune.
